# EUROPEAN PATENT SPECIFICATION

(11) **EP 1 367 121 B1**
(45) Date of publication and mention of the grant of the patent: **19.05.2010**
(21) Application number: 03253252.5
(22) Date of filing: 23.05.2003
(51) Int. Cl.: C12N 11/12

(54) **Process for immobilizing polypeptides via the carboxyl group of the C-terminus**
Prozess zur Immobilisierung von Polypeptiden über die Carboxylgruppe des C-terminus
Procédé d'immobilisation des polypéptides par le groupe carboxylique du C-terminus

(30) Priority: 23.05.2002 JP 2002148950
(43) Date of publication of application: 03.12.2003
(73) Proprietor: National Institute of Advanced Industrial Science and Technology, Tokyo 100-8921 (JP)
(72) Inventor: Iwakura, Masahiro, Ushiku-shi, Ibaraki 300-1206 (JP); Hirota, Kiyonori, Ibaraki 305-0067 (JP)
(74) Representative: Woods, Geoffrey Corlett

(56) References cited:
- DATABASE WPI Section Ch, Week 200051 Derwent Publications Ltd., London, GB; Class C07,Page 15, AN 2000-444747 XP002256965 -& JP 2000 247999 A (AGENCY OF IND SCIENCE & TECHNOLOGY) 12 September 2000 (2000-09-12)
- DATABASE WPI Section Ch, Week 200031 Derwent Publications Ltd., London, GB; Class C07,Page 10, AN 2000-056223 XP002256966 -& JP 2000 119300 A (AGENCY OF IND SCI & TECHNOLOGY) 25 April 2000 (2000-04-25)
- DATABASE WPI Section Ch, Week 199615 Derwent Publications Ltd., London, GB; Class C12,Page 10, AN 1996-145943 XP002256967 -& JP 08 033485 A (AGENCY OF IND SCI & TECHNOLOGY) 6 February 1996 (1996-02-06)
- DATABASE WPI Section Ch, Week 199817 Derwent Publications Ltd., London, GB; Class C07,Page 7, AN 1998-189301 XP002256968 -& JP 10 045798 A (AGENCY OF IND SCI & TECHNOLOGY) 17 February 1998 (1998-02-17)
- IWAKURA MASAHIRO ET AL: "Immobilization of dihydrofolate reductase by engineered cysteine residue attached to its C-terminal end" JOURNAL OF BIOCHEMISTRY (TOKYO), vol. 114, no. 3, 1993, pages 339-343, XP009018698 ISSN: 0021-924X

### SEQUENCE LISTING

<110> Institute of Advanced Industrial Science and Technology
<120> Process for immobilizing orientation-controlled protein and process for arraying and immobilizing protein using the same
<130> N.88841 GCW
<140>
   <141>
<160> 11
<170> PatentIn Ver. 2.1
<210> 1
   <211> 239
   <212> PRT
   <213> Artificial Sequence
<220>
   <223> Description of Artificial Sequence: Artificial sequence
<400> 1
   Met Ala Ser Lys Gly Glu Glu Leu Phe Thr Gly Val Val Pro Ile Leu
   1 5 10 15
   Val Glu Leu Asp Gly Asp Val Asn Gly His Lys Phe Ser Val Ser Gly
   20 25 30
   Glu Gly Glu Gly Asp Ala Thr Tyr Gly Lys Leu Thr Leu Lys Phe Ile
   35 40 45
   Cys Thr Thr Gly Lys Leu Pro Val Pro Trp Pro Thr Leu Val Thr Thr
   50 55 60
   Leu Cys Tyr Gly Val Gln Cys Phe Ser Arg Tyr Pro Asp His Met Lys
   65 70 75 80
   Arg His Asp Phe Phe Lys Ser Ala Met Pro Glu Gly Tyr Val Gln Glu
   85 90 95
   Arg Thr Ile Phe Phe Lys Asp Asp Gly Asn Tyr Lys Thr Arg Ala Glu
   100 105 110
   Val Lys Phe Glu Gly Asp Thr Leu Val Asn Arg Ile Glu Leu Lys Gly
   115 120 125
   Ile Asp Phe Lys Glu Asp Gly Asn Ile Leu Gly His Lys Leu Glu Tyr
   130 135 140
   Asn Tyr Asn Ser His Asn Val Tyr Ile Met Ala Asp Lys Gln Lys Asn
   145 150 155 160
   Gly Ile Lys Val Asn Phe Lys Thr Arg His Asn Ile Glu Asp Gly Ser
   165 170 175
   Val Gln Leu Ala Asp His Tyr Gln Gln Asn Thr Pro Ile Gly Asp Gly
   180 185 190
   Pro Val Leu Leu Pro Asp Asn His Tyr Leu Ser Thr Gln Ser Ala Leu
   195 200 205
   Ser Lys Asp Pro Asn Glu Lys Arg Asp His Met Val Leu Leu Glu Phe
   210 215 220
   Val Thr Ala Ala Gly Ile Thr His Gly Met Asp Glu Leu Tyr Asn
   225 230 235
<210> 2
   <211> 253
   <212> PRT
   <213> Artificial Sequence
<220>
   <223> Description of Artificial Sequence: Artificial sequence
<400> 2
   Met Ala Ser Lys Gly Glu Glu Leu Phe Thr Gly Val Val Pro Ile Leu
   1               5                   10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Leu Cys Tyr Gly Val Gln Cys Phe Ser Arg Tyr Pro Asp His Met Lys
   65                  70                  75                  80
   Arg His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Thr Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Thr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr His Gly Met Asp Glu Leu Tyr Asn Gly
   225                 230                 235                 240
   Gly Gly Gly Gly Gly Cys Ala Asp Asp Asp Asp Asp Asp
                   245                 250
<210> 3
   <211> 239
   <212> PRT
   <213> Artificial Sequence
<220>
   <223> Description of Artificial Sequence: Artificial, sequence
<400> 3
   Met Ala Ser Lys Gly Glu Glu Leu Phe Thr Gly Val Val Pro Ile Leu
   1 5 10 15
   Val Glu Leu Asp Gly Asp Val Asn Gly His Lys Phe Ser Val Ser Gly
   20 25 30
   Glu Gly Glu Gly Asp Ala Thr Tyr Gly Lys Leu Thr Leu Lys Phe Ile
   35 40 45
   Cys Thr Thr Gly Lys Leu Pro Val Pro Trp Pro Thr Leu Val Thr Thr
   50 55 60
   Leu Ser His Gly Val Gln Cys Phe Ser Arg Tyr Pro Asp His Met Lys
   65 70 75 80
   Arg His Asp Phe Phe Lys Ser Ala Met Pro Glu Gly Tyr Val Gln Glu
   85 90 95
   Arg Thr Ile Phe Phe Lys Asp Asp Gly Asn Tyr Lys Thr Arg Ala Glu
   100 105 110
   Val Lys Phe Glu Gly Asp Thr Leu Val Asn Arg Ile Glu Leu Lys Gly
   115 120 125
   Ile Asp Phe Lys Glu Asp Gly Asn Ile Leu Gly His Lys Leu Glu Tyr
   130 135 140
   Asn Tyr Asn Ser His Asn Val Tyr Ile Met Ala Asp Lys Gln Lys Asn
   145 150 155 160
   Gly Ile Lys Ala Asn Phe Lys Ile Arg His Asn Ile Glu Asp Gly Ser
   165 170 175
   Val Gln Leu Ala Asp His Tyr Gln Gln Asn Thr Pro Ile Gly Asp Gly
   180 185 190
   Pro Val Leu Leu Pro Asp Asn His Tyr Leu Ser Thr Gln Ser Ala Leu
   195 200 205
   Ser Lys Asp Pro Asn Glu Lys Arg Asp His Met Val Leu Leu Glu Phe
   210 215 220
   Val Thr Ala Ala Gly Ile Thr His Gly Met Asp Glu Leu Tyr Lys
   225 230 235
<210> 4
   <211> 253
   <212> PRT
   <213> Artificial Sequence
<220>
   <223> Description of Artificial Sequence: Artificial sequence
<400> 4
   Met Ala Ser Lys Gly Glu Glu Leu Phe Thr Gly Val Val Pro Ile Leu
   1               5                   10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Leu Ser His Gly Val Gln Cys Phe Ser Arg Tyr Pro Asp His Met Lys
   65                  70                  75                  80
   Arg His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Ala Asn Phe Lys Ile Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Thr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr His Gly Met Asp Glu Leu Tyr Lys Gly
   225                 230                 235                 240
   Gly Gly Gly Gly Gly Cys Ala Asp Asp Asp Asp Asp Asp
                   245                 250
<210> 5
   <211> 239
   <212> PRT
   <213> Artificial Sequence
<220>
   <223> Description of Artificial Sequence: Artificial sequence
<400> 5
   Met Val Ser Lys Gly Glu Glu Leu Phe Thr Gly Val Val Pro Ile Leu
   1               5                   10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Phe Gly Tyr Gly Leu Gln Cys Phe Ala Arg Tyr Pro Asp His Met Lys
   65                  70                  75                  80
   Gln His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Ile Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Tyr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr Leu Gly Met Asp Glu Leu Tyr Lys
   225                 230                 235
<210> 6
   <211> 253
   <212> PRT
   <213> Artificial Sequence
<220>
   <223> Description of Artificial Sequence: Artificial sequence
<400> 6
   Met Val Ser Lys Gly Glu Glu Leu Phe Thr Gly Val Val Pro Ile Leu
    1               5                  10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Phe Gly Tyr Gly Leu Gln Cys Phe Ala Arg Tyr Pro Asp His Met Lys
   65                  70                  75                  80
   Gln His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Ile Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Tyr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr Leu Gly Met Asp Glu Leu Tyr Lys Gly
   225                 230                 235                 240
   Gly Gly Gly Gly Gly Cys Ala Asp Asp Asp Asp Asp Asp
                   245                 250
<210> 7
   <211> 225
   <212> PRT
   <213> Artificial Sequence
<220>
   <223> Description of Artificial Sequence: Artificial sequence
<400> 7
   Met Ala Ser Ser Glu Asn Val Ile Thr Glu Phe Met Arg Phe Lys Val
    1               5                  10                  15
   Arg Met Glu Gly Thr Val Asn Gly His Glu Phe Glu Ile Glu Gly Glu
               20                  25                  30
   Gly Glu Gly Arg Pro Tyr Glu Gly His Asn Thr Val Lys Leu Lys Val
           35                  40                  45
   Thr Lys Gly Gly Pro Leu Pro Phe Ala Trp Asp Ile Leu Ser Pro Gln
       50                  55                  60
   Phe Gln Tyr Gly Ser Lys Val Tyr Val Lys His Pro Ala Asp Ile Pro
   65                  70                  75                  80
   Asp Tyr Lys Lys Leu Ser Phe Pro Glu Gly Phe Lys Trp Glu Arg Val
                   85                  90                  95
   Met Asn Phe Glu Asp Gly Gly Val Ala Thr Val Thr Gln Asp Ser Ser
               100                 105                 110
   Leu Gln Asp Gly Cys Phe Ile Tyr Lys Val Lys Phe Ile Gly Val Asn
           115                 120                 125
   Phe Pro Ser Asp Gly Pro Val Met Gln Lys Lys Thr Met Gly Trp Glu
       130                 135                 140
   Ala Ser Thr Glu Arg Leu Tyr Pro Arg Asp Gly Val Leu Lys Gly Glu
   145                 150                 155                 160
   Thr His Lys Ala Leu Lys Leu Lys Asp Gly Gly His Tyr Leu Val Glu
                   165                 170                 175
   Phe Lys Ser Ile Tyr Met Ala Lys Lys Pro Val Gln Leu Pro Gly Tyr
               180                 185                 190
   Tyr Tyr Val Asp Ala Lys Leu Asp Ile Thr Ser His Asn Glu Asp Tyr
           195                 200                 205
   Thr Ile Val Glu Gln Tyr Glu Arg Thr Glu Gly Arg His His Leu Phe
       210                 215                 220
   Leu
   225
<210> 8
   <211> 239
   <212> PRT
   <213> Artificial Sequence
<220>
   <223> Description of Artificial Sequence: Artificial sequence
<400> 8
   Met Ala Ser Ser Glu Asn Val Ile Thr Glu Phe Met Arg Phe Lys Val
   1 5 10 15
   Arg Met Glu Gly Thr Val Asn Gly His Glu Phe Glu Ile Glu Gly Glu
   20 25 30
   Gly Glu Gly Arg Pro Tyr Glu Gly His Asn Thr Val Lys Leu Lys Val
   35 40 45
   Thr Lys Gly Gly Pro Leu Pro Phe Ala Trp Asp Ile Leu Ser Pro Gln
   50 55 60
   Phe Gln Tyr Gly Ser Lys Val Tyr Val Lys His Pro Ala Asp Ile Pro
   65 70 75 80
   Asp Tyr Lys Lys Leu Ser Phe Pro Glu Gly Phe Lys Trp Glu Arg Val
   85 90 95
   Met Asn Phe Glu Asp Gly Gly Val Ala Thr Val Thr Gln Asp Ser Ser
   100 105 110
   Leu Gln Asp Gly Cys Phe Ile Tyr Lys Val Lys Phe Ile Gly Val Asn
   115 120 125
   Phe Pro Ser Asp Gly Pro Val Met Gln Lys Lys Thr Met Gly Trp Glu
   130 135 140
   Ala Ser Thr Glu Arg Leu Tyr Pro Arg Asp Gly Val Leu Lys Gly Glu
   145 150 155 160
   Thr His Lys Ala Leu Lys Leu Lys Asp Gly Gly His Tyr Leu Val Glu
   165 170 175
   Phe Lys Ser Ile Tyr Met Ala Lys Lys Pro Val Gln Leu Pro Gly Tyr
   180 185 190
   Tyr Tyr Val Asp Ala Lys Leu Asp Ile Thr Ser His Asn Glu Asp Tyr
   195 200 205
   Thr Ile Val Glu Gln Tyr Glu Arg Thr Glu Gly Arg His His Leu Phe
   210 215 220
   Leu Gly Gly Gly Gly Gly Gly Cys Ala Asp Asp Asp Asp Asp Asp
   225 230 235
<210> 9
   <211> 171
   <212> PRT
   <213> Artificial Sequence
<220>
   <223> Description of Artificial Sequence: Artificial sequence
<400> 9
   Met Ile Ser Leu Ile Ala Ala Leu Ala Val Asp Arg Val Ile Gly Met
    1               5                  10                  15
   Glu Asn Ala Met Pro Trp Asn Leu Pro Ala Asp Leu Ala Trp Phe Lys
               20                  25                  30
   Arg Asn Thr Leu Asn Lys Pro Val Ile Met Gly Arg His Thr Trp Glu
           35                  40                  45
   Ser Ile Gly Arg Pro Leu Pro Gly Arg Lys Asn Ile Ile Leu Ser Ser
       50                  55                  60
   Gln Pro Gly Thr Asp Asp Arg Val Thr Trp Val Lys Ser Val Asp Glu
   65                  70                  75                  80
   Ala Ile Ala Ala Ala Gly Asp Val Pro Glu Ile Met Val Ile Gly Gly
                   85                  90                  95
   Gly Arg Val Tyr Glu Gln Phe Leu Pro Lys Ala Gln Lys Leu Tyr Leu
               100                 105                 110
   Thr His Ile Asp Ala Glu Val Glu Gly Asp Thr His Phe Pro Asp Tyr
           115                 120                 125
   Glu Pro Asp Asp Trp Glu Ser Val Phe Ser Glu Phe His Asp Ala Asp
       130                 135                 140
   Ala Gln Asn Ser His Ser Tyr Ser Phe Glu Ile Leu Glu Arg Arg Gly
   145                 150                 155                 160
   Gly Gly Gly Cys Ala Asp Asp Asp Asp Asp Asp
                   165                 170
<210> 10
   <211> 197
   <212> PRT
   <213> Artificial Sequence
<220>
   <223> Description of Artificial Sequence: Artificial sequence
<400> 10
   Ala Ser Thr Asp Tyr Trp Gln Asn Trp Thr Asp Gly Gly Gly Ile Val
   1               5                   10                  15
   Asn Ala Val Asn Gly Ser Gly Gly Asn Tyr Ser Val Asn Trp Ser Asn
               20                  25                  30
   Thr Gly Asn Phe Val Val Gly Lys Gly Trp Thr Thr Gly Ser Pro Phe
           35                  40                  45
   Arg Thr Ile Asn Tyr Asn Ala Gly Val Trp Ala Pro Asn Gly Asn Gly
       50                  55                  60
   Tyr Leu Thr Leu Tyr Gly Trp Thr Arg Ser Pro Leu Ile Glu Tyr Tyr
   65                  70                  75                  80
   Val Val Asp Ser Trp Gly Thr Tyr Arg Pro Thr Gly Thr Tyr Lys Gly
                   85                  90                  95
   Thr Val Lys Ser Asp Gly Gly Thr Tyr Asp Ile Tyr Thr Thr Thr Arg
               100                 105                 110
   Tyr Asn Ala Pro Ser Ile Asp Gly Asp Arg Thr Thr Phe Thr Gln Tyr
           115                 120                 125
   Trp Ser Val Arg Gln Ser Lys Arg Pro Thr Gly Ser Asn Ala Thr Ile
       130                 135                 140
   Thr Phe Ser Asn His Val Asn Ala Trp Lys Ser His Gly Met Asn Leu
   145                 150                 155                 160
   Gly Ser Asn Trp Ala Tyr Gln Val Met Ala Thr Glu Gly Tyr Gln Ser
                   165                 170                 175
   Ser Gly Ser Ser Asn Val Thr Val Trp Gly Gly Gly Gly Cys Ala Asp
               180                 185                 190
   Asp Asp Asp Asp Asp
           195
<210> 11
   <211> 360
   <212> PRT
   <213> Artificial Sequence
<220>
   <223> Description of Artificial Sequence: Artificial sequence
<400> 11
   Ala Ser Thr Asp Tyr Trp Gln Asn Trp Thr Asp Gly Gly Gly Ile Val
   1               5                   10                  15
   Asn Ala Val Asn Gly Ser Gly Gly Asn Tyr Ser Val Asn Trp Ser Asn
               20                  25                  30
   Thr Gly Asn Phe Val Val Gly Lys Gly Trp Thr Thr Gly Ser Pro Phe
           35                  40                  45
   Arg Thr Ile Asn Tyr Asn Ala Gly Val Trp Ala Pro Asn Gly Asn Gly
       50                  55                  60
   Tyr Leu Thr Leu Tyr Gly Trp Thr Arg Ser Pro Leu Ile Glu Tyr Tyr
   65                  70                  75                  80
   Val Val Asp Ser Trp Gly Thr Tyr Arg Pro Thr Gly Thr Tyr Lys Gly
                   85                  90                  95
   Thr Val Lys Ser Asp Gly Gly Thr Tyr Asp Ile Tyr Thr Thr Thr Arg
               100                 105                 110
   Tyr Asn Ala Pro Ser Ile Asp Gly Asp Arg Thr Thr Phe Thr Gln Tyr
           115                 120                 125
   Trp Ser Val Arg Gln Ser Lys Arg Pro Thr Gly Ser Asn Ala Thr Ile
       130                 135                 140
   Thr Phe Ser Asn His Val Asn Ala Trp Lys Ser His Gly Met Asn Leu
   145                 150                 155                 160
   Gly Ser Asn Trp Ala Tyr Gln Val Met Ala Thr Glu Gly Tyr Gln Ser
                   165                 170                 175
   Ser Gly Ser Ser Asn Val Thr Val Trp Gly Gly Gly Gly Met Ile Ser
               180                 185                 190
   Leu Ile Ala Ala Leu Ala Val Asp Arg Val Ile Gly Met Glu Asn Ala
           195                 200                 205
   Met Pro Trp Asn Leu Pro Ala Asp Leu Ala Trp Phe Lys Arg Asn Thr
       210                 215                 220
   Leu Asn Lys Pro Val Ile Met Gly Arg His Thr Trp Glu Ser Ile Gly
   225                 230                 235                 240
   Arg Pro Leu Pro Gly Arg Lys Asn Ile Ile Leu Ser Ser Gln Pro Gly
                   245                 250                 255
   Thr Asp Asp Arg Val Thr Trp Val Lys Ser Val Asp Glu Ala Ile Ala
               260                 265                 270
   Ala Ala Gly Asp Val Pro Glu Ile Met Val Ile Gly Gly Gly Arg Val
           275                 280                 285
   Tyr Glu Gln Phe Leu Pro Lys Ala Gln Lys Leu Tyr Leu Thr His Ile
       290                 295                 300
   Asp Ala Glu Val Glu Gly Asp Thr His Phe Pro Asp Tyr Glu Pro Asp
   305                 310                 315                 320
   Asp Trp Glu Ser Val Phe Ser Glu Phe His Asp Ala Asp Ala Gln Asn
                   325                 330                 335
   Ser His Ser Tyr Ser Phe Glu Ile Leu Glu Arg Arg Gly Gly Gly Gly
               340                 345                 350
   Cys Ala Asp Asp Asp Asp Asp Asp
           355                 360

## Description

### Technical Field

The present invention discloses an immobilized protein. The present invention further discloses an orientation-controlled immobilized protein. More specifically, the present invention discloses effective production of an immobilized protein that is immobilized only at the carboxy terminus. Further, the present invention discloses a process for producing a protein array having an immobilized protein with a controlled orientation while being arrayed on a carrier for immobilization (a substrate or a basal plate).

### Background Art

It has been heretofore attempted to use a soluble protein as an immobilized protein by binding it with, for example, an insoluble carrier such as agarose gel. Examples of such attempts are the development of an immobilized enzyme prepared by binding an enzyme protein to an insoluble carrier and the production of an enzyme reactor utilizing the same.

In immobilizing enzymes, chemical binding to an insoluble carrier has been mainly carried out by utilizing the reactivity of the amino acid side chain, which constitutes a protein. For example, a cysteine residue has an SH group as a functional group, and disulfidation, alkylation, acylation, etc. are known as the reactions of the SH group. Utilization of these reactions enables the immobilization of proteins via a side chain of the cysteine residue. A lysine residue has an amino group as a side chain. This amino group can form an amide bond with a carboxyl group using carbodiimide. Similarly, aspartic acid and glutamic acid have carboxyl groups, and thus, they can form an amide bond with a primary amine using carbodiimide. However, the immobilization reaction utilizing a functional group of a side chain has some problems. That is to say, for example, the reaction relies on the amino acid sequence of a protein, the immobilized site cannot be specified since a protein contains several amino acids of the same kind, and the possibility of immobilization at several sites cannot be eliminated.

In order to solve the aforementioned problems, a reaction for immobilizing the protein via a carboxyl group of the carboxy terminus of the protein utilizing the amino bond forming reaction via a cyanocysteine residue was developed (see JP Patent Publication (Kokai) No. 10-45798), and a means for binding a protein via one location at the carboxy terminus and via a main chain was developed (see Japanese Patent No. 3047020). Binding of a protein via one location at the carboxy terminus and via a main chain enhanced the reversibility of denaturation and brought advantages, such as the ability to produce an immobilized enzyme that enables heat sterilization of an immobilized protein.

The amide bond forming reaction via a cyanocysteine residue, however, is represented by reaction formula (a): NH₂-R-CO-NH-CH(CH₂-SCN)-CO-X + NH₂-B → NH₂-R-CO-NH-B (wherein R represents a chain of arbitrary amino acid residues, X represents OH, an arbitrary amino acid residue, or a chain of arbitrary amino acid residues, and NH₂-B represents an arbitrary primary amine compound).

This amide bond formation reaction occurs competitively with the reaction between the cleavage reaction of a peptide chain represented by reaction formula (b): NH₂-R-CO-NH-CH(CH₂-SCN)-CO-X + H₂O → NH₂-R-COOH + Z (wherein R represents a chain of arbitrary amino acid residues, X represents OH, an arbitrary amino acid residue, or a chain of arbitrary amino acid residues, and Z represents a 2-iminothiazoline-4-carboxylyl derivative of X) (see G. R. Jacobson, M. H. Schaffer, G. R. Stark, T. C. Vanaman, J. Biological Chemistry, 248, 6583-6591 (1973)) and a reaction in which a thiocyano group is eliminated and a cyanocysteine residue is converted into dehydroalanine in the β-elimination reaction (see Y. Degani, A. Patchomik, Biochemistry, 13,1-11 (1974)) represented by reaction formula (c): NH₂-R-CO-NH-CH(CH₂-SCN)-CO-X → NH₂-R-CO-NH-C(CH₂)-CO-X (wherein R represents a chain of an arbitrary amino acid residue, and X represents OH, an arbitrary amino acid residue, or a chain of an arbitrary amino acid residue). Thus, a problem occurred in terms of reaction yield. Specifically, while protein immobilization using reaction formula (a) has an advantage, that is, a protein can bind via one location at the carboxy terminus and via a main chain, the problem of immobilization yield was an issue of concern.

In order to solve this problem, a process has been developed in which a peptide, which has on its carboxy terminus a peptide sequence having a cyanocysteine residue and a negative charge around the neutral region, and a protein to be immobilized are allowed to react with a carrier for immobilization having a primary amine, thereby effectively producing an immobilized protein (see Japanese Patent No. 3047020).

However, in the case of an immobilization reaction wherein a cysteine residue in a linker peptide chain is previously cyanated followed by adsorption via the ion interaction between the peptide sequence exhibiting a negative charge that was introduced in the linker peptide chain and a base plate, the cleavage reaction by cyanated cysteine and the side reaction of dihydroalanine generation are increased with the elapse of time after the formation of cyanated cysteine. This deteriorated the immobilization reaction efficiency. Further, it was impossible to produce homogenous enzyme reactors.

### Summary of the Invention

An object of the present invention is to provide a process for binding a protein to a carrier by controlling its orientation and a protein binding carrier comprising a protein bound to the carrier while its orientation is controlled. More specifically, the present invention provides a process for binding a protein to a carrier by controlling its orientation utilizing the amide bond formation reaction via cyanocysteine, wherein a protein, which is prepared by fusing a protein containing a cysteine residue having a sulfhydryl group to a protein to be immobilized, is allowed to be adsorbed on a carrier for immobilization, the sulfhydryl group is cyanated using a cyanating reagent, and the protein of interest is then immobilized. Further, the present invention enables the protein to be previously arrayed and adsorbed on the carrier utilizing, for example, the principle of ink jet printers. Accordingly, it is an object of the present invention to provide a process for obtaining a protein array by binding a protein to a carrier by controlling its orientation and a protein array comprising a protein immobilized thereon while its orientation is controlled.

The present inventors have conducted concentrated studies regarding a process for binding a protein to a carrier for immobilization while maintaining the functionality of the protein. As a result, they have found that a protein can be effectively bound to a carrier for immobilization while its orientation is controlled by preparing a protein, which is prepared by binding a peptide sequence having a sulfhydryl group and a negative charge around the neutral region to the protein to be immobilized, adsorbing the protein onto the carrier for immobilization, and cyanating the sulfhydryl group. This has led to the completion of the present invention.

More specifically, the present invention provides the following:
[1] a process for immobilizing on a carrier for immobilization a protein represented by general formula (1):

   NH₂-R₁-COOH (1)

   wherein a protein having a sulfhydryl group represented by general formula (2) is produced:

   NH₂R₁-CONH-R₂-CO-NH-CH(CH₂-SH)-CO-NH-R₃-COOH (2),

   the resulting protein is immobilized adsorptively on the carrier for immobilization represented by general formula (3) via ion interactions under neutral conditions:

   NH₂-Y (3), and

   a sulfhydryl group of the cysteine residue in the protein represented by general formula (2) that is adsorbed on the carrier for immobilization represented by general formula (3) is cyanated using a cyanating reagent to convert it into a cyanocysteine residue,
   thereby producing an immobilized protein represented by general formula (4):

   NH₂-R₁-CO-NH-R₂-CO-NH-Y (4)

   wherein R₁ and R₂ represent arbitrary amino acid sequences, R₃ represents an amino acid sequence that has a strong negative charge around the neutral region and can acidify the isoelectric point of NH₂-R₁-CONH-R₂-CO-NH-CH(CH₂-SH)-CO-NH-R₃-COOH, and Y represents an arbitrary carrier for immobilization;
[2] the process for producing an immobilized protein according to [1] above, wherein the protein represented by general formula (2) has a sulfhydryl group represented by NH₂-R₁-CONH-R₂-CO-NH-CH(CH₂-SH)-CO-NH-CH(CH₃)-CO-[NH-CH(CH₂-COOH)-CO]ₙ-OH wherein R₁ and R₂ represent arbitrary amino acid sequences and n is a natural number,
[3] the process for producing an immobilized protein according to [1] or [2] above, wherein R₂ in the protein represented by general formula (2) is polyglycine;
[4] the process for producing an immobilized protein according to any one of [1] to [3] above, wherein R₁ in the protein represented by general formula (2) is an enzyme;
[5] a process for producing a protein array by immobilizing on a substrate for immobilization a protein represented by general formula (1):

   NH₂-R₁-COOH (1),

   wherein a protein having a sulfhydryl group represented by general formula (2) is produced:

   NH₂-R₁-CONH-R₂-CO-NH-CH(CH₂SH)-CO-NH-R₃-COOH (2),

   the resulting protein is immobilized adsorptively on the substrate for immobilization represented by general formula (3) while arraying:

   NH₂-Y (3),

   and
   a sulfhydryl group of the cysteine residue in the protein represented by general formula (2) that is adsorbed on the substrate for immobilization represented by general formula (3) is cyanated using a cyanating reagent to convert it into a cyanocysteine residue,
   thereby producing a protein array having an immobilized protein represented by general formula (4):

   NH₂-R₁-CO-NH-R₂-CO-NH-Y (4)

   wherein R₁ and R₂ represent arbitrary amino acid sequences, R₃ represents an amino acid sequence that has a strong negative charge around the neutral region and can acidify the isoelectric point of NH₂-R₁-CONH-R₂-CO-NH-CH(CH₂-SH)-CO-NH-R₃-COOH, and Y represents an arbitrary substrate for immobilization;
[6] the process for producing a protein array according to [5] above, wherein the process for adsorbing the protein on a substrate for immobilization while arraying is carried out by an ink jet system;
[7] the process for producing a protein array according to [6] above, wherein a cyanating reagent, which cyanates a sulfhydryl group of the cysteine residue, is allowed to act using an ink jet printer;
[8] the process for producing a protein array according to any one of [5] to [7] above, wherein the protein represented by general formula (2) has a sulfhydryl group represented by NH₂-R₁-CONH-R₂-CO-NH-CH(CH₂-SH)-CO-NH-CH(CH₃)-CO-[NH-CH(CH₂-COOH-CO]ₙ-OH wherein R₁ and R₂ represent arbitrary amino acid sequences and n is a natural numbers.

### Brief description of the Drawings

Fig. 1 is a diagram showing the correlation between the amount of the inputted protein and the total amount of the immobilized protein.
Fig. 2 is a diagram showing the correlation between the amount of the inputted protein and the ratio (%) of the immobilized protein.
Fig. 3 is a diagram showing the fluorescence emission spectrum of a green fluorescent protein that is immobilized on Amino-Cellulofine.
Fig. 4 is a diagram showing the correlation between a length of time of standing after cyanation and the ratio of the immobilized protein when immobilization was carried out using a previously cyanated green fluorescent protein.

### Preferred embodiment of the present invention

The present invention is hereafter described in detail.

### 1. Adsorptive immobilization of protein by the process according to the present invention

According to the process of immobilizing an orientation-controlled protein of the present invention, a protein can be effectively immobilized on a carrier while maintaining the activity thereof. Proteins to be immobilized are not limited, and various proteins can be immobilized depending on the use. For example, an enzyme can be immobilized to produce an enzyme reactor, a specific intermolecular interaction of the immobilized protein can be utilized to produce a carrier for separation, or a specific antigen or antibody can be immobilized to produce a carrier for diagnosis, which comprises the antigen or antibody immobilized thereon and utilizes an antigen-antibody reaction. Also, an arbitrary protein can be immobilized to produce an analysis tool for proteomics, etc.

In the present invention, the term "immobilization" is defined as the fixation of a substance by binding it to a carrier for immobilization. This includes binding caused by a covalent bond, binding caused by electrostatic interaction which does not result from a covalent bond, and the like. In order to distinguish the state where a substance is immobilized adsorptively on a carrier from the state where a substance is immobilized by covalent bond, the former condition is referred to as "adsorption" or "adsorptive immobilization" in the following description.

In order to implement the present invention, a protein represented by general formula (2); NH₂-R₁-CO-NH-R₂-CO-NH-CH(CH₂-SH)-CO-NH-R₃-COOH should be first prepared in order to immobilize the protein represented by general formula (1): NH₂-R₁-COOH. In these general formulae, R₁ and R₂ represent arbitrary amino acid sequences and R₃ represents a chain of an arbitrary amino acid residue, which has a strong negative charge around the neutral region and can acidify the isoelectric point of NH₂-R₁-CO-NH-R₂-CO-NH-CH(CH₂-SH)-CO-NH-R₃-COOH. R₂ is a linker peptide between a protein to be immobilized, which is represented by general formula (1), and a carrier. R₂ is arbitrary and the type and the number of amino acids are not limited. R₂ is preferably (Gly)ₓ, wherein x is from 1 to 30, preferably from 2 to 20, for example from 2 to 10 or for example 2, 3, 4 or 5. For example, Gly-Gly-Gly-Gly can be used. A gene encoding a protein represented by general formula (1) is bound to a gene encoding a peptide sequence represented by general formula (5): NH₂-R₂-CO-NH-CH(CH₂-SH)-CO-NH-R₃-COOH (wherein R₂ and R₃ are as defined above) to prepare a gene encoding a fusion protein represented bu general formula (2): NH₂-R₁-CO-NH-R₂-CO-NH-CH(CH₂-SH)-CO-NH-R₃-COOH. The resulting gene is allowed to express itself in a host organism such as *E*. *coli*, and the expressed protein is then separated and purified. Thus, a protein of interest can be obtained. Such a fusion protein can be prepared utilizing conventional techniques (see, for example, M. Iwakura et al., J. Biochem. 111:37-45 (1992)). Alternatively, the fusion protein can be also prepared by combining a technique of genetic engineering and a conventional technique of protein synthesis, or by a technique of protein synthesis alone.

In general formulae (2) and (5), R₃ is preferably a sequence containing many aspartic acids or glutamic acids. The isoelectric point of the protein depends on the type and number of the amino acids that constitute the protein. For example, when the protein contains many basic amino acids such as lysine or arginine, quantities of aspartic acids or glutamic acids should be large enough to exceed the total number of basic amino acids. The isoelectric point of the protein can be easily deduced by calculation by those skilled in the art. Preferably, a sequence may be designed to contain may aspartic acids or glutamic acids, thereby setting the isoelectric point of the substance represented by general formula (2) between 4 and 5.

An example of a suitable sequence is alanyl-polyaspartic acid. This is because the substitution of the amino acid subsequent to cyanocysteine with alanine can induce easy amide bond forming reactions via a cyanocysteine residue, and because a carboxyl group of aspartic acid is the most acidic in the amino acid side chain. R₃ may be a negatively charged amino acid sequence comprising Ala(Asp)_{y} or Ala(Glu)_{y}, wherein y is from 1 to 30, preferably from 2 to 20, for example from 2 to 10 or for example 2, 3, 4, 5 or 6.

The thus obtained protein represented by general formula (2) is immobilized adsorptively on a carrier for immobilization. Any insoluble carrier having primary amino groups can be used as the carrier for immobilizations represented by general formula (3): NH₂-Y used in the present invention. Any form of carrier for immobilizations, such as gel, bead, particulate, or plate carriers can be used. Examples of commercially available carriers having primary amino groups that can be used include Amino-Cellulofine (available from SEIKAGAKU CORPORATION), AF-Amino-Toyopearl (available from TOSOH), EAH-Sepharose 4B and Lysine-Sepharose 4B (available from Amersham Pharmacia), AffiGel 102 (available from BioRad), and Porous 20 NH (available from Boehringer Mannheim). Also, a silane compound having a primary amine (e.g., 3-aminopropylmethoxysilane) is used to introduce the primary amine onto glass beads, glass plates, and the like, and the resultant can be used as a carrier.

A protein having a sulfhydryl group represented by general formula (2), i.e., NH₂-R₁-CO-NH-R₂-CO-NH-CH(CH₂-SH)-CO-NH-R₃-COOH, and a carrier for immobilization represented by general formula (3), i.e., NH₂-Y, can be reacted under neutral to weak alkaline conditions (pH 7 to 10) at room temperature. Under weak alkaline reaction conditions, the protein represented by general formula (2) has a negative charge, the carrier for immobilization represented by general formula (3) has a positive charge, and they are bound adsorptively to each other by the electrostatic interaction. Thus, this reaction can be utilized as a capture reaction for the amide bond forming reaction represented by the reaction formula (a) as presented above. Since this electrostatic interaction depends on the salt concentration in a solvent, preferably, a solvent to be used has as low a salt concentration as possible. For example, a 10 mM phosphate buffer (pH 7 to 9) can be used, although any salt concentration may be adopted as long as the electrostatic interaction is suitably carried out.

Accordingly, any solution can be used as a solution for the adsorption reaction as long as the solution is a solvent that can assure the electrostatic interaction, that can dissolve the protein represented by general formula (2), and that can adjust the pH level. In addition to various buffers such as a phosphate buffer or a borate buffer, and alcohols such as methanol or ethanol, dimethylformamide, dimethyl sulfoxide, or the like can be used. Although high reaction efficiency can be yielded at room temperature, any temperature range may be adopted without causing difficulties as long as the solvent used is not frozen or boiled and the protein represented by general formula (2) does not aggregate as a result of denaturation.

The protein represented by general formula (2) is bound adsorptively to the carrier represented by general formula (3): NH₂-Y via the electrostatic interaction in the aforementioned step. The strength of the bond can be varied depending on changes in salt concentration and the like. As long as the strong bond can be maintained, the protein represented by general formula (6): NH₂-R₁-CONH-R₂-CO-NH-CH(CH₂-SH)-CO-NH-R₃-COOH(-)-(+)NH₂-Y (wherein (-)--(+) represents a state of being bound adsorptively via an ionic bond) that is adsorbed on the carrier for immobilization can be used for various applications. For example, if a protein is an enzyme, a carrier for immobilization can be used as an enzyme reactor. When gel, particulate, or bead-like substances are used as carriers for immobilizations, these substances can be effectively used as enzyme reactors by, for example, putting them into a suitable support such as a column. If a protein is a specific antigen or antibody, it can be used as a device for diagnosis utilizing an antigen-andbody reaction. A protein adsorbed on the carrier for immobilization represented by general formula (2) can be easily desorbed by treating it with a highly concentrated salt solution such as 1M KCl, and can be recovered as NH₂-R₁-CONH-R₂-CO-NH-CH(CH₂-SH)-CO-NH-R₃-COOH. The use of this property enables the recovery of a specific substance by adsorbing and immobilizing a protein that can interact with a specific substance on a carrier, bringing the protein into contact with a solution containing the specific substance, and then desorbing the adsorbed immobilized protein from the carrier, thereby recovering the specific substance in a state that is bound to the adsorbed and immobilized protein. The substance can be then separated from the protein, thereby obtaining the substance in isolation. Any combination of a protein and a substance that can interact with each other may be used, such as an antibody and an antigen, a ligand and a receptor, a protein and another protein such as an enzyme and a substrate, or a protein and a non-protein. The utilization of this technique enables efficient concentration and separation of a specific substance. Further, cyanation of a protein that is bound adsorptively to a carrier for immobilization represented by general formula (6): NH₂-R₁-CONH-R₂-CO-NH-CH(CH₂-SH)-CO-NH-R₃-COOH(-)--(+)NH₂-Y in the following manner allows the protein to more strongly bind covalently to a carrier. Thus, a stable protein-carrier for immobilization can be obtained. Specifically, the carrier having a protein immobilized adsorptively thereon represented by general formula (6) can be used as an intermediate for producing an immobilized protein having a strongly immobilized protein by covalent bond represented by general formula (4): NH₂-R₁-CO-NH-R₂-CO-NH-Y.

The protein represented by general formula (2) is immobilized adsorptively on a carrier for immobilization to perform cyanation. Thus, an immobilized protein that is immobilized by a covalent bond on the carrier for immobilization represented by general formula (4) can be prepared. In this case, a sulfhydryl group of a cysteine residue in a linker peptide chain, which was introduced into a protein sequence adsorbed on the carrier for immobilization, is cyanated, and binding occurs between amino acid residues immediately before the cyanated cysteine residue.

Cyanation can be performed using a cyanating reagent. A simple process generally utilizes 2-nitro-5-thiocyanobennzoic acid (NTCB) (see Y. Degani, A. Ptchornik, Biochemistry, 13,1-11 (1974)) or 1-cyano-4-dimethylaminopyridinium tetrafluoroborate (CDAP) as a cyanating reagent. Commercially available NTCB and CDAP can be used. Cyanation using NTCB can be efficiently carried out at pH levels between 7 and 9, and the reaction efficiency can be investigated based on an increase in the absorbance at 412 nm of free thionitrobenzoic acid (the molecular extinction coefficient = 13,600 M¹cm⁻¹). Cyanation of the SH group can be carried out also in accordance with the process described in the literature (see J. Wood & Catsipoolas, J. Biol. Chem. 233, 2887 (1963)). Cyanation using a cyanating reagent may be carried out after the adsorptive immobilization of the protein represented by general formula (2): NH₂-R₁-CO-NH-R₂-CO-NH-CH(CH₂-SH)-CO-NH-R₃-COOH on the carrier for immobilization. Alternatively, it may be carried out simultaneously with the adsorptive immobilization. In the case of the latter, the protein represented by general formula (2) and a cyanating reagent may be simultaneously applied to the carrier for immobilization.

The reaction involving cyanocysteine used in the present invention can cause hydrolysis as a side reaction. All the reaction products that are generated by such a possible side reaction dissolve in a solvent, and thus, side reaction products can be removed by washing the carrier for immobilization with a suitable solvent after the reaction. Accordingly, all the immobilized proteins prepared by immobilization according to the present invention are represented by general formula (4): NH₂-R₁-CO-NH-R₂-CO-NH-Y (wherein R₁, R₂, and Y are as defined above) and bound to the carrier for immobilization via one location at the carboxy terminus of the protein of interest.

A characteristic of the thus obtained immobilized protein is that a carboxy terminus is bound to a carrier via only one location. This can effectively exhibit functions of the protein. For example, when an enzyme protein having a catalytic function is used as an immobilized protein, the catalytic function is lost once the protein is denatured by raising temperature or adding a denaturing agent. The immobilized enzyme prepared by the immobilization process according to the present invention can fully regain its functions by removing denaturing conditions.

In accordance with the process according to the present invention, the protein represented by general formula (1) can be immobilized at a high efficiency of at least 80%, and almost 100% of functions of the immobilized protein can be sustained. By this process, the protein can be stably and strongly immobilized on the carrier for immobilization. If a protein to be immobilized is an enzyme, a carrier for immobilization can be used as an enzyme reactor. When gel, particulate, or bead-like substances are used as carriers for immobilization, these substances can be efficiently used as enzyme reactors by, for example, putting them into suitable supports such as columns. When a protein is a specific antigen or antibody, it can be used as a device for diagnosis and the like that utilize the antigen-antibody reaction.

The amino group in a carrier having a primary amino group represented by general formula (3): NH₂-Y that was not used for immobilization can be masked using a low molecular weight peptide having a sequence represented by general formula (2). For example, a peptide comprising a sequence of alanine-cysteine-aspartic acid is chemically synthesized as the low molecular weight peptide corresponding to general formula (2), and the alanine-cysteine-aspartic acid is again immobilized adsorptively on the immobilized protein carrier represented by general formula (4): NH₂-R₁-CO-NH-R₂-CO-NH-Y, followed by cyanation. Thus, an alanine residue can be bound to the unreacted primary amine.

A summary of processes for immobilizing a protein according to the present invention is hereafter provided.

Adding a sequence for immobilization by gene modification of a protein to be immobilized

Obtaining a modified protein (expression, separation, purification)

Adsorbing on a primary amine carrier via ion interactions

Cyanating the SH group of cysteine of adsorbed protein

Immobilizing by peptide bond forming reaction via cyanocysteine

Desorbing an unimmobilized protein from a carrier

Masking a free primary amine in a carrier

Obtaining an orientation-controlled immobilized protein

Among these, processes of adsorbing on a primary amine carrier via ion interactions and cyanating the SH group of cysteine of the adsorbed protein are important for the production of a protein array that is hereafter described in detail.

### 2. Production of a protein array by immobilizing while arraying

The process for immobilizing a protein of the present invention can be used to produce a protein array by immobilizing a protein on a substrate or basal plate (hereinafter referred to as a "substrate") while arraying the protein. In the present invention, a protein array comprises at least one protein being arrayed and immobilized on a substrate such as glass, a nylon membrane, etc. at high density. It is also referred to as a "protein chip." Proteins are immobilized at an extremely high density in the protein array, and thus, a small amount of protein should be immobilized efficiently with its orientation being controlled. Accordingly, the process according to the present invention wherein a protein can be immobilized with its orientation being controlled in a highly efficient manner is suitable for producing a protein array.

In order to immobilize the protein while arraying it by the process according to the present invention, a protein having a sulfhydryl group represented by general formula (2): NH₂-R₁-CONH-R₂-CO-NH-CH(CH₂-SH)-CO-NH-R₃-COOH is first arrayed and adsorbed on a sheet carrier for immobilization (substrate) represented by general formula (4): NH₂-Y. A substrate for immobilizing a protein that is used for producing a protein array is not limited. Any substrate can be used as long as the substrate is a sheet onto which the NH₂ group has been introduced. The aforementioned carrier, a nylon membrane, a glass plate, etc. to which an amino group has been introduced in the aforementioned manner can be used as a carrier.

Any arraying process can be used without limitation, as long as a protein solution can be arrayed on a substrate at high density. For example, an arrayer that spots a protein solution on a substrate may be used. The arrayer that spots a protein solution may use various means such as a pin, quill pen, ink jet, capillary, or pin and ring, and any process may be adopted. Alternatively, a picking robot may be used. The protein array generally immobilizes the protein solution on a substrate by dot-spotting. As described below, for example, a protein solution can be immobilized in line by an ink jet system. In the present invention, when one or more proteins are arrayed on a substrate, it is referred to as a protein array, regardless of its shape.

The protein having a sulfhydryl group represented by general formula (2): NH₂-R₁-CONH-R₂-CO-NH-CH(CH₂-SH)-CO-NH-R₃-COOH is arrayed on the substrate for immobilization represented by general formula (4): NH₂-Y under neutral to weak alkaline conditions (pH 7 to 10) by various processes such as those mentioned above. Thus, a protein array comprising the protein R₁ adsorbed on a substrate by a formula NH₂R₁-CONH-R₂-CO-NH-CH(CH₂-SH)-CO-NH-R₃-COOH(-)--(+)-NH₂-Y (wherein (-)-(+) represents a bond by adsorption via an ionic bond) can be obtained. Further, the adsorbed protein represented by this formula can be treated with a cyanating reagent to obtain a protein array comprising the protein R₁ represented by a formula NH₂-R₁-CO-NH-R₂-CO-NH-Y arrayed and immobilized on a substrate via a covalent bond. Cyanation can be carried out with the addition of a cyanating agent to the surface of the substrate having a protein adsorbed thereon. Alternatively, cyanation can be carried out by, for example, immersing a whole substrate having a protein adsorbed thereon in a cyanating agent. Also, a protein is allowed to adsorb by the aforementioned arraying means, and a cyanating reagent may be then applied to the substrate on the portion having a protein adsorbed thereon by a similar arraying means. For example, a protein is adsorbed and arrayed on a substrate with a constant dot pattern using a pin, and a cyanating reagent solution instead of a protein is spotted using a pin in such a manner that the cyanating agent is superposed on a protein. This generates cyanation, and the protein is thereby immobilized. A protein represented by general formula (2): NH₂-R₁-CONH-R₂-CO-NH-CH(CH₂-SH)-CO-NH-R₃-COOH and a cyanating agent may be simultaneously applied to the substrate for immobilization.

Arraying by the inkjet system is hereafter described in detail as one example of arraying.

The protein for use in the present invention can be immobilized utilizing ink jet printing techniques, thereby producing a protein array.

During the step of immobilizing the protein, the protein represented by general formula (2): NH₂-R₁-CO-NH-R₂-CO-NH-CH(CH₂-SH)-CO-NH-R₃-COOH is immobilized adsorptively on a suitable substrate having an amino group introduced therein represented by NH₂-Y by ink jet printing techniques. Subsequently, a sulfhydryl group of the cysteine residue in the protein is cyanated to convert it into a cyanocysteine residue. Thus, a protein array having the immobilized protein represented by general formula (4): NH₂-R₁-CO-NH-R₂-CO-NH-Y can be produced.

Ink jet printers used in printing are classified according to their ink ejection systems. One type is a piezo system, in which an element (piezo element), which is deformed by the application of voltage, is used, an ink storage space in the head is reduced by the deformation, and inks are ejected by this pressure. Another type is a thermal ink jet system, in which a heater in the nozzle is heated to generate foams, and inks are pushed out by these foams. Either type of printer can be used for the immobilization according to the present invention. A piezo system printer is preferable when denaturation of proteins by heat is taken into consideration. Commercially available printers can be used as ink jet printers, and examples thereof include ink jet printers of the EPSON PM Series.

The protein solution represented by general formula (2) may be filled into a ink cartridge of a printer and then installed in the printer. Printing patterns of the printer can be freely determined with the use of a suitable software such as a drawing software. A line pattern may be adopted, which immobilizes the protein solution in line or a dot pattern as an arbitrary number of dots in a given area. Also, the amount of the protein solution discharged during a single operation can be freely determined. For example, when the line pattern is adopted, the protein solution may be immobilized in line with a width of several dozen µm to several hundred µm. If the amount and the rate of discharge are suitably determined, the protein solution can be immobilized with a line pattern having an arbitrary width within the determined range. When the dot pattern is adopted, a drop of protein solution is discharged relative to a dot, and a protein can be immobilized as a dot of a circle having a diameter of several µm to several hundred µm. Alternatively, patterning at the time of printing can be adjusted to form a rectangular dot measuring several µm to several hundred µm per side. In this case, the amount and the rate of discharging the protein solution may be suitably determined depending on a desired dot pattern.

Thus, the protein represented by general formula (2) is immobilized adsorptively on a substrate and treated with a cyanating agent, thereby obtaining the substrate for immobilization having the protein represented by general formula (4) immobilized adsorptively thereon. Cyanation can be carried out by treating a whole substrate having the protein represented by general formula (2) immobilized adsorptively thereon with a cyanating reagent. In this case, a cyanating reagent may be added to the whole surface of the substrate, or the whole substrate may be immersed in the cyanating reagent. Alternatively, a cyanating reagent may be applied to the carrier on its portion having a protein immobilized thereon after the adsorptive immobilization of the protein represented by general formula (2) using an ink jet printer on the carrier. In this case, after the adsorptive immobilization of the protein, a cartridge is replaced with one filled with a cyanating reagent, and a cyanating reagent may be applied in the same pattern as with the adsorptive immobilization of the protein.

The protein that is arrayed and immobilized using ink jet printing techniques and the like can be evaluated in terms of its immobilization pattern and status using an electron microscope, an electrochemical microscope, or other means.

The present invention is hereafter described with reference to the following examples, although the technical scope of the present invention is not limited to these examples.

### EXAMPLES

In the present examples, immobilization was carried out using a dihydrofolate reductase, a xylanase, a fusion protein of xylanase-dihydrofolate reductase, and various fluorescent proteins. All the proteins that were used for the immobilization were prepared as follows. A gene encoding the amino acid sequence of the protein corresponding to general formula (1) was obtained, a gene encoding an amino acid sequence prepared by binding 8 to 10 amino acid sequences on the carboxy terminal side to the amino acid sequence represented by general formula (5) was chemically synthesized, the resulting gene was chemically synthesized with a portion of a gene encoding 8 to 10 amino acid sequences on the amino terminal side of the protein represented by general formula (1), and the resultant was used as a primer for PCR and amplified by PCR. Thus, a gene encoding the amino acid sequence corresponding to general formula (2) was prepared, the gene was incorporated into an expression vector, and then expressed in *E*. *coli*, followed by separation and purification. Those skilled in the art can easily produce the protein represented by general formula (2) that is subjected to the immobilization according to the present invention if they can obtain a gene encoding the protein represented by general formula (1).

In the present examples, proteins that were used as those corresponding to general formula (1) were a dihydrofolate reductase (amino acids 1 to 159 in SEQ ID NO: 9), a xylanase (amino acids 1 to 185 in SEQ ID NO: 10), a fusion protein of xylanase-dihydrofolate reductase (amino acids 1 to 348 in SEQ ID NO: 11), and fluorescent proteins exhibiting various types of fluorescence (four types, SEQ ID NOs: 1, 3, 5, and 7). Each of the full-length sequences of SEQ ID NOs: 9, 10, and 11 corresponds to the protein represented by general formula (2). Regarding genes encoding a dihydrofolate reductase, a xylanase, and a fusion protein of xylanase-dihydrofolate reductase, those produced by Iwakura (one of the present inventors) were used. Regarding fluorescent proteins, commercially available ones were used. Fluorescent proteins emitting green fluorescence (GFP) (SEQ ID NO: 1) and blue fluorescence (BFP) (SEQ ID NO: 3) were purchased from Quantum, and genes encoding fluorescent proteins emitting yellow fluorescence (YFP) (SEQ ID NO: 5) and red fluorescence (DsRed) (SEQ ID NO: 7) were purchased from Clontech. It should be clearly noted that the present invention is not limited by processes for obtaining genes.

### [Example 1]

### Examination of the immobilization reaction using a green fluorescent protein

A green fluorescent protein (GFP) (SEQ ID NO: 1) was used as the protein corresponding to general formula (1) NH₂-R₁-COOH to produce a protein to be immobilized (SEQ ID NO: 2) corresponding to general formula (2). Thus, conditions for the immobilization reaction, etc. were examined.

The fusion protein was immobilized using Amino-Cellulofine (purchased from SEIKAGAKU CORPORATION), and Amino-Toyopearl (purchased from TOSOH) was used as a primary amine carrier.

Immobilization was carried out in the following manner.

Each protein had previously been dialyzed with a 10 mM phosphate buffer (pH 8.0) containing a 1000-fold amount of 5 mM ethylenediaminetetraacetic acid (EDTA) three times or more, and the dialyzed protein sample was diluted with a buffer, which was the same as the one used in the dialysis. Thus, protein samples at various concentrations were prepared.

The thus prepared protein to be immobilized (50 µl) was mixed with 50 µl of Amino-Cellulofine (amine content: 20 µmoles NH₂/ml) or Amino-Toyopearl (primary amine content: 130 µmoles NH₂/ml), and the mixture was mildly stirred at room temperature for at least 2 hours. Thereafter, the mixture was centrifuged at 1000 rpm for several seconds to submerge the carrier, the supernatant was taken out, and the fluorescence intensity was measured. This value was compared with the fluorescence intensity of the protein sample that was first inputted to determine the rate of successfully adsorbed proteins. When the amount of the inputted proteins is not more than 100 nmoles per ml of a carrier, all the inputted proteins (100%) were immobilized adsorptively on the carrier. The proteins that were immobilized adsorptively in this state could be completely released from the carrier by treatment with a 10 mM phosphate buffer (pH 8.0) containing 1M KCI. This strongly suggests that the immobilization at this phase depends on the ion interactions between the positive charge of the primary amine of the carrier and the negative charge of polyaspartic acid, which was introduced into the protein to be immobilized.

The SH group of cysteine of the adsorbed protein was cyanated by suspending the carrier comprising the protein adsorptively immobilized thereon in a 10 mM phosphate buffer (pH 7.0) containing 5 mM EDTA, adding 2-nitro-5-thiocyanobenzoic acid (NTCB) to a final concentration of 5 mM, and allowing the reaction to proceed at room temperature for 4 hours. Thereafter, centrifugation was carried out at 1000 rpm for several seconds, the carrier was submerged, and the supernatant was removed, followed by suspension in a 10 mM phosphate buffer (pH 7.0). This procedure was repeated 5 times to eliminate NTCB, etc.

The cyanated adsorptively immobilized protein was centrifuged at 1000 rpm for several seconds, the carrier was submerged, the supernatant was removed, and the protein was suspended in a 10 mM borate buffer (pH 9.5) containing 5 mM EDTA, followed by mild stirring at room temperature for at least 24 hours. Thus, the immobilization reaction was carried out. Thereafter, centrifugation was carried out at 1000 rpm for several seconds, the carrier was submerged, and the supernatant was removed, followed by suspension in a 10 mM phosphate buffer (pH 8.0) containing 1M KCl. This procedure was repeated 5 times to eliminate a side reaction product of the immobilization.

Through the above procedure, the immobilized protein represented by general formula (4), the orientation of which is controlled, and which has the main chain at the carboxy terminus of the protein bound thereto via an amide bond, was obtained.

Similar results were obtained by performing cyanation under the same conditions, except for the use of 1-cyano-4-dimethylaminopyridinium tetrafluoroborate (CDAP) as a cyanating reagent instead of NTCB.

The correlation between the amount of the inputted protein and the amount of the immobilized protein was investigated in the following manner.

Reaction of a dihydrofolate reductase, a xylanase, and a fusion protein of dihydrofolate reductase-xylanase were monitored by measuring the fluorescence emission spectra in the ultraviolet regions (excited at 290 nm), and reactions of various fluorescent proteins were monitored by measuring the fluorescence emission spectra in the visible regions of the proteins.

The fluorescence emission spectrum was measured using a spectrofluorophotometer (FP-750, JASCO). The protein fluorescence in the solution was measured at 25°C by dissolving the fluorescent protein in 3 ml of 10 mM phosphate buffer (pH 8.0) and placing the protein solution in a cuvette for the fluorescence measurement. The fluorescence emission spectrum of the fluorescent protein bound to the carrier was measured while constantly stirring the carrier in a cuvette under the same conditions as those pertaining when the measurement of the protein in the solution was conducted.

The spectrum of the fluorescent protein shifts toward the long wavelength side by being adsorptively or covalently bound to the carrier. Accordingly, the ratio of adsorptive immobilization in the adsorption process (the ratio (B/A) of the amount of the adsorptively immobilized protein (B) relative to the amount of the inputted protein (A)) and the ratio of immobilization in the immobilization process (the ratio (E/D) of the amount of the finally immobilized protein (E) relative to the amount of the adsorptively immobilized protein (D)) were separately determined. The ratio of the finally immobilized protein relative to the inputted protein is represented by (B/A) x (E/D), using the determined values. Since the fluorescence emission spectrum shifts in the process of adsorptive immobilization, the fluorescence emission spectrum in the inputted protein solution and the fluorescence emission spectrum of the supernatant after the adsorption reaction were measured, the amount of the protein remaining unadsorbed (this is designated as "C") was measured, and the value of B above was determined by calculating as B = A - C and used in the measurement of the ratio of adsorptive immobilization in the process of adsorptive immobilization. The fluorescence emission spectrum was measured using a suspension of a carrier having a protein immobilized thereon. The background, which was derived from a carrier having no protein immobilized thereon, was corrected. This corrected value was used for measuring D and E.

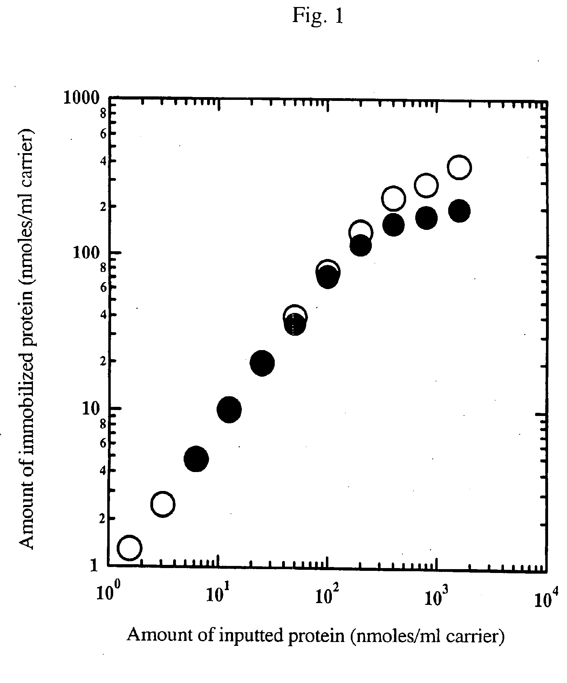

The results are shown in Figs. 1 to 3.

Fig. 1 is a diagram showing the correlation between the amount of the inputted protein and the total amount of the immobilized protein, wherein a white circle indicates Amino-Cellulofine and a black circle indicates Amino-Toyopearl. As shown in the drawing, when the amount of the inputted protein was up to about 400 nmoles per ml of the carrier, the amount of the immobilized protein increased in a manner approximately proportional to the amount of the inputted protein. If the amount exceeded the aforementioned range, the immobilization peaked out.

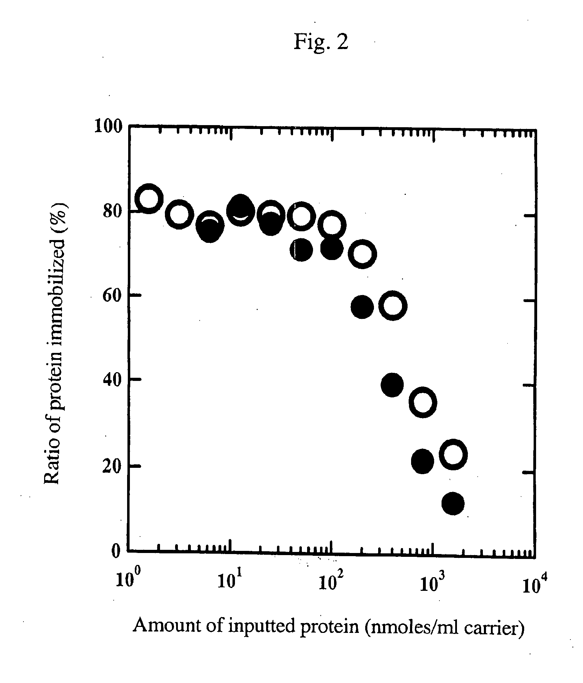

Fig. 2 is a diagram showing the correlation between the amount of the inputted protein and the ratio (%) of the immobilized protein, wherein a white circle indicates Amino-Cellulofine and a black circle indicates Ammo-Toyopearl. As shown in the drawing, when the amount of the inputted protein was not more than about 100 nmoles per ml of the carrier, the ratio of the immobilized protein was approximately constant at about 80%. If the amount exceeded the aforementioned range, the yield factor of the immobilization gradually deteriorated.

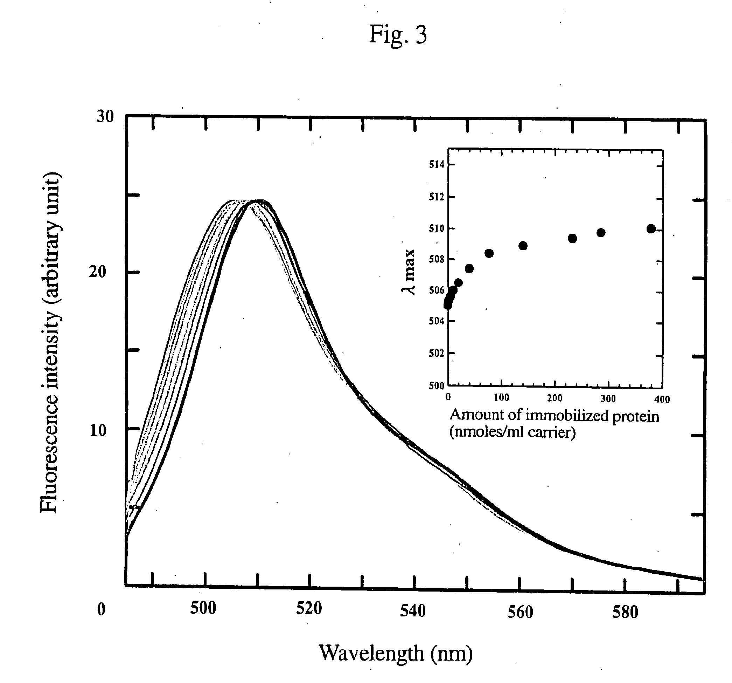

Fig. 3 is a diagram showing the fluorescence emission spectrum of a green fluorescent protein immobilized on Amino-Cellulofine. The diagram that is inserted in Fig. 3 shows the correlation between the amount of the immobilized protein and the wavelength (λmax) exhibiting the maximal fluorescence intensity. The fluorescence emission spectrum of a green fluorescent protein immobilized on Amino-Cellulofine was predisposed to shift toward the long wavelength side as the amount of immobilization per carrier increased. This dependence of the fluorescence emission spectrum on the amount of the immobilized protein exhibits biphase, i.e., it is considered to reflect the effect obtained by a decrease in an average distance between immobilized proteins and the effect obtained by a decrease in the degree of freedom in the movement of the protein immobilized at a high density.

The immobilized green fluorescent protein exhibits a fluorescence emission spectrum caused by the fluorescent resonance energy transfer (FRET) as the amount of immobilization per unit of catalyst increases. This indicates that the protein is immobilized in an orientation-controlled manner, and immobilization at high density with a controlled orientation can be achieved.

### [Example 2]

Comparison of functionalities between the immobilized protein obtained by cyanation after adsorption on a carrier and the immobilized protein obtained by cyanation followed by adsorption on a carrier

Immobilization was carried out in two ways, i.e., a conventional process wherein a green fluorescent protein is cyanated before being adsorbed on the carrier for immobilization, and the process of the present invention wherein the protein to be immobilized represented by general formula (2) is adsorbed on the carrier for immobilization, followed by cyanation. Thus, the efficiencies of immobilization for the both processes were compared and evaluated.

A process of cyanating before adsorption was carried out in the following manner according to the process described in Japanese Patent No. 3047020.

In a 0.1M tris hydrochloride buffer (pH 7.4) containing 5 mM ethylenediaminetetraacetic acid (EDTA), a fivefold amount of 2-nitro-5-thiocyanobenzoic acid (NTCB) was added to the green fluorescent protein, and the reaction was allowed to proceed at room temperature for 4 hours. Thus, cyanation was carried out. Based on an increase in the absorbance of free thionitrobenzoic acid at 412 nm (a molecular extinction coefficient of 13,600 M¹cm¹), it was confirmed that the cysteine residue was approximately quantitatively cyanated. Unreacted NTCB and thionitrobenzoic acid were removed from the cyanation reaction solution by gel filtration using the Sephadex G-50 column. A 10 mM borate buffer (pH 9.5) containing 5 mM EDTA was used as an eluent. The cyanated protein sample in this state was used in the immobilization reaction.

The correlation between the amount of the inputted protein and the amount of the immobilized protein was investigated in the same manner as the process described in Example 1.

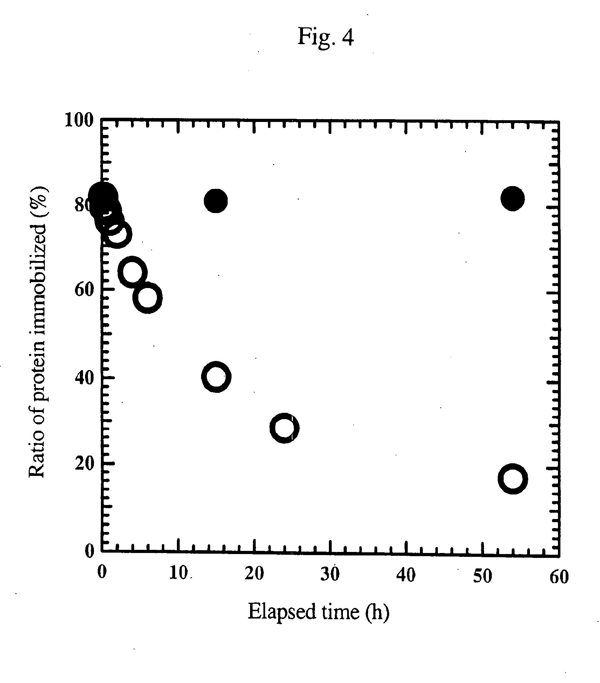

The results are shown in Fig. 4.

Fig. 4 is a diagram showing the correlation between the length of time of standing after cyanation and the ratio of the protein immobilized when immobilization was carried out using a previously cyanated green fluorescent protein. In Fig. 4, a white circle indicates a result obtained when a previously cyanated green fluorescent protein was used and a black circle indicates a result of the process according to the present invention.

In a process for improving the efficiency of immobilization wherein a cysteine residue in a linker peptide chain has been previously cyanated, and the adsorption is then realized via the ion interaction between a peptide sequence exhibiting a negative charge that was introduced into the linker peptide chain and a substrate, i.e:, in a conventional process, a cleavage reaction by cyanated cysteine and a side reaction of a dihydroalanine producing reaction increase with the elapse of time after the formation of cyanated cysteine. Thus, the efficiency of the immobilization deteriorates. In accordance with the process of the present invention, however, this deterioration in efficiency can be completely prevented.

### [Example 3]

### Dependence of the immobilization efficiency on a protein to be immobilized

A dihydrofolate reductase (SEQ ID NO: 9), a xylanase (SEQ ID NO: 10), a fusion protein of xylanase-dihydrofolate reductase (SEQ ID NO: 11), a green fluorescent protein (GFP) (SEQ ID NO: 2), a blue fluorescent protein (BFP) (SEQ ID NO: 4), a yellow fluorescent protein (YFP) (SEQ ID NO: 6), and a red fluorescent protein variant (DsRed) (SEQ ID NO: 8) were used as the proteins represented by general formula (1), and Amino-Cellulofine was used as a carrier for immobilization to perform immobilization by the process according to the present invention. The immobilization efficiency was then measured. The sequence identification numbers indicate amino acid sequences of the proteins corresponding to general formula (5), wherein each protein was fused with a protein having a sulfhydryl group.

The aforementioned protein was immobilized using Amino-Cellulofine as a carrier in the same manner as described in Example 1.

Table 1 shows the maximal immobilization efficiency for each of the proteins.

**Table 1**

| Amino-Cellutofine | |
|---|---|
| Proteins to be immobilized | Maximal ratio of immobilized protein (%) |
| Dihydrofolate reductase | > 75 |
| Xylanase | > 75 |
| Fusion protein of xylanase-dihydrofolate reductase | > 75 |
| GFP | 81.4 |
| BFP | 88.2 |
| YFP | 76.2 |
| DsRed | 90.4 |

Values widely vary per measurement because of the conditions of up to 10 nmoles/ml of carrier except for the case of GFP, and because of the fluorescence measurement at the ultraviolet region except for fluorescent proteins. However, values exceeding 75% were obtained for all measurements.

### [Example 3]

### Dependence of the immobilization efficiency on the carrier for immobilization

Commercially available bead carriers for immobilization, Amino-Cellulofine, Amino-Toyopearl, AH Sepharose, and Polyallylamine Beads were used as carriers for immobilization, and a green fluorescent protein (GFP) was used as a protein to be immobilized to perform immobilization. Immobilization was carried out in the same manner as described in Example 1. The ratio of immobilization was investigated in the same manner as described in Example 1.

Table 2 shows the maximal immobilization efficiency for each of the carriers.

**Table 2**

| Measurement using GFP | |
|---|---|
| Kind of carrier for immobilization | Maximal ratio of immobilized protein (%) |
| Amino-Cellulofine | 81.4 |
| Amino-Toyopearl | 82.9 |
| AffiGel 102 | 82.8 |
| EAH-Sepharose 4B | 66.5 |
| Porous 20 NH. | 76.8 |

Except for Amino-Cellulofine and Amino-Toyopearl, values were obtained under conditions of up to 10 nmole/ml of carrier.

The results shown in Table 2 indicate that the protein can be immobilized on any carrier, as long as the carrier has a primary amine.

### Effect of the Invention

According to a process for immobilizing a protein of the present invention, a protein can be immobilized in high yiled with its orientation being controlled. As shown in Example 2, a protein can be immobilized more effectively by the method of the present invention, compared to the conventional method wherein a protein is adsorbed on a carrier for immobilization after being cyanated.

Further, the method of the present invention which utilizes an ink jet printer can immobilize a large amount of various proteins within a short period of time, and thus, a protein array can be efficiently produced.

## Claims

1. A process for immobilizing on a carrier for immobilization a protein represented by general formula (1):
NH₂-R₁-COOH (1)
wherein a protein having a sulfhydryl group represented by general formula (2) is produced:
NH₂-R₁-CONH-R₂-CO-NH-CH(CH₂-SH)-CO-NH-R₃-COOH (2)
the resulting protein is immobilized adsorptively on the carrier for immobilization represented by general formula (3) via ion interactions under neutral conditions:
NH₂-Y (3),
and
a sulfhydryl group of the cysteine residue in the protein represented by general formula (2) that is adsorbed on the carrier for immobilization represented by general formula (3) is cyanated using a cyanating reagent to convert it into a cyanocysteine residue,
thereby producing an immobilized protein represented by general formula (4):
NH₂-R₁-CO-NH-R₂-CO-NH-Y (4)
wherein R₁ and R₂ represent arbitrary amino acid sequences, R₃ represents an amino acid sequence that has a strong negative charge around the neutral region and can acidify the isoelectric point of NH₂-R₁-CONH-R₂-CO-NH-CH(CH₂-SH)-CO-NH-R₃-COOH, and Y represents an arbitrary carrier for immobilization.

2. The process for producing an immobilized protein according to claim 1, wherein the protein represented by general formula (2) has a sulfhydryl group represented by NH₁-R₁-CONH-R₂-CO-NH-CH(CH₂-SH)-CO-NH-CH(CH₃)-CO-[NH-CH(CH₂-COOH)-CO]ₙ-OH wherein R₁ and R₂ represent arbitrary amino acid sequences and n is a natural number.

3. The process for producing an immobilized protein according to claim 1 or 2, wherein R₂ in the protein represented by general formula (2) is polyglycine.

4. The process for producing an immobilized protein according to any one of claims 1 to 3, wherein R₁ in the protein represented by general formula (2) is an enzyme.

5. A process for producing a protein array by immobilizing on a substrate for immobilization a protein represented by formula (1):
NH₂-R₁-COOH (1),
wherein a protein having a sulfhydryl group represented by general formula (2) is produced:
NH₂-R₁-CONH-R₂-CO-NH-CH(CH₂-SH)-CO-NH-R₃-COOH (2),
the resulting protein is immobilized adsorptively on the substrate for immobilization represented by general formula (3) while arraying:
NH₂-Y (3),
and
a sulfhydryl group of the cysteine residue in the protein represented by general formula (2) that is adsorbed on the carrier for immobilization represented by general formula (3) is cyanated using a cyanating reagent to convert it into a cyanocysteine residue,
thereby producing a protein array having an immobilized protein represented by general formula (4):
NH₂-R₁-CO-NH-R₂-CO-NH-Y (4)
wherein R₁ and R₂ represent arbitrary amino acid sequences, R₃ represents an amino acid sequence that has a strong negative charge around the neutral region and can acidify the isoelectric point of NH₂-R₁-CONE-R₂-CO-NH-CH(CH₂-SH)-CO-NH-R₃-COOH, and Y represents an arbitrary substrate for immobilization.

6. The process for producing a protein array according to claim 5, wherein the process for adsorbing the protein on a substrate for immobilization while arraying is carried out by an ink jet system.

7. The process for producing a protein array according to claim 6, wherein a cyanating reagent, which cyanates a sulfhydryl group of the cysteine residue, is allowed to act using an ink jet printer.

8. The process for producing a protein array according to any one of claims 5 to 7, wherein the protein represented by general formula (2) has a sulfhydryl group represented by NH₂-R₁-CONH-R₂-CO-NH-CH(CH₂-SH)-CO-NH-CH(CH₃)-CO-[NH-CH(CH₂-COOH)-CO]ₙ-OH wherein R₁ and R₂ represent arbitrary amino acid sequences and n is a natural number.

## Patentansprüche

1. Verfahren für die Immobilisierung auf einem Träger, zur Immobilisierung eines Proteins, dargestellt durch die allgemeine Formel (1):
NH₂-R₁-COOH (1)
wobei ein Protein mit einer Sulfhydrylgruppe, dargestellt durch die allgemeine Formel (2) erzeugt wird:
NH₂-R₁-CONH-R₂-CO-NH-CH(CH₂-SH)-CO-NH-R₃-COOH (2)
wobei das resultierende Protein adsorptiv auf den Träger zur Immobilisierung, dargestellt durch die allgemeine Formel (3) über Ioneninteraktionen unter neutralen Bedingungen immobilisiert wird:
NH₂-Y (3)
und
wobei eine Sulfhydrylgruppe des Cysteinrests in dem Protein, dargestellt durch die allgemeine Formel (2), adsorbiert auf dem Träger für die Immobilisierung, dargestellt durch die allgemeine Formel (3) unter Verwendung eines Cyanierungsreagenzes cyaniert wird um sie in einen Cyanocysteinrest umzuwandeln,
wodurch ein immobilisiertes Protein erzeugt wird, dargestellt durch die allgemeine Formel (4) :
NH₂-R₁-CO-NH-R₂-CO-NH-Y (4)
worin R₁ und R₂ willkürliche Aminosäuresequenzen repräsentieren, R₃ bedeutet eine Aminosäuresequenz, die eine starke negative Ladung um die neutrale Region aufweist und den isoelektrischen Punkt von NH₂-R₁-CONH-R₂-CO-NH-CH(CH₂-SH)-CO-NH-R₃-COOH ansäuern kann und Y bedeutet einen willkürlichen Träger für die Immobilisierung.

2. Verfahren zur Erzeugung eines immobilisierten Proteins gemäß Anspruch 1, wobei das durch die allgemeine Formel (2) dargestellte Protein eine Sulfhydrylgruppe aufweist, dargestellt durch NH₂-R₁-CONH-R₂-CO-NH-CH(CH₂-SH)-CO-NH-CH(CH₃)-CO-[NH-CH(CH₂-COOH)-CO]ₙ-OH, worin R₁ und R₂ willkürliche Aminosäuresequenzen darstellen und n eine natürliche Zahl ist.

3. Verfahren zur Erzeugung eines immobilisierten Proteins gemäß Anspruch 1 oder 2, worin R₂ in dem durch die allgemeine Formel (2) dargestellten Protein Polyglycin ist.

4. Verfahren zur Erzeugung eines immobilisierten Proteins gemäß einem der Ansprüche 1 bis 3, worin R₁ in dem durch die allgemeine Formel (2) dargestellten Protein ein Enzym ist.

5. Verfahren zur Erzeugung eines Proteinarrays durch Immobilisierung auf einem Substrat für die Immobilisierung eines durch die Formel (1) dargestellten Proteins:
NH₂-R₁-COOH (1)
wobei ein Protein mit einer Sulfhydrylgruppe, dargestellt durch die allgemeine Formel (2) erzeugt wird:
NH₂-R₁-CONH-R₂-CO-NH-CH(CH₂-SH)-CO-NH-R₃-COOH (2)
wobei das resultierende Protein adsorptiv auf dem Substrat für die Immobilisierung, dargestellt durch die allgemeine Formel (3) immobilisiert wird, während der Array durchgeführt wird:
NH₂-Y (3)
und
eine Sulfhydrylgruppe des Cysteinrests in dem Protein, das durch die allgemeine Formel (2) dargestellt wird, adsorbiert auf dem Träger für die Immobilisierung, wie dargestellt durch die allgemeine Formel (3), unter Verwendung eines Cyanierungsreagenzes cyaniert wird, um sie in einen Cyanocysteinrest umzuwandeln,
wodurch ein Proteinarray erzeugt wird, der ein immobilisiertes Protein aufweist, dargestellt durch die allgemeine Formel (4):
NH₂-R₁-CO-NH-R₂-CO-NH-Y (4)
worin R₁ und R₂ willkürliche Aminosäuresequenzen darstellen, R₃ stellt eine Aminosäuresequenz dar, die eine stark negative Ladung um den neutralen Bereich aufweist und den isoelektrischen Punkt von NH₂-R₁-CONH-R₂-CO-NH-CH(CH₂-SH)-CO-NH-R₃-COOH ansäuern kann und Y stellt ein willkürliches Substrat für die Immobilisierung dar.

6. Verfahren zur Erzeugung eines Proteinarrays gemäß Anspruch 5, wobei das Verfahren zur Adsorption des Proteins auf einem Substrat für die Immobilisierung während der Arraydurchführung durch ein Tintenstrahlsystem durchgeführt wird.

7. Verfahren zur Erzeugung eines Proteinarrays gemäß Anspruch 6, wobei es einem Cyanierungsreagenz, das eine Sulfhydrylgruppe des Cysteinrests cyaniert, ermöglicht wird, zu wirken, wobei ein Tintenstrahldrucker verwendet wird.

8. Verfahren zur Erzeugung eines Proteinarrays gemäß einem der Ansprüche 5 bis 7, wobei das durch die allgemeine Formel (2) dargestellte Protein eine Sulfhydrylgruppe aufweist, dargestellt durch NH₂-R₁-CONH-R₂-CO-NH-CH(CH₂-SH)-CO-NH-CH(CH₃)-CO-[NH-CH(CH₂-COOH)-CO]n-OH, worin R₁ und R₂ willkürliche Aminosäuresequenzen darstellen und n eine natürliche Zahl ist.

## Revendications

1. Procédé pour immobiliser, sur un support d'immobilisation, une protéine représentée par la formule générale (1) :
NH₂-R₁-COOH (1)
dans laquelle une protéine comportant un groupe sulfhydryle représenté par la formule générale (2) est produite :
NH₂-R₁-CONH-R₂-CO-NH-CH(CH₂-SH)-CO-NH-R₃-COOH (2)
la protéine résultante est immobilisée par adsorption sur le support d'immobilisation représenté par la formule générale (3) via des interactions ioniques dans des conditions neutres :
NH₂-Y (3),
et
un groupe sulfhydryle du résidu cystéine dans la protéine représentée par la formule générale (2) qui a été adsorbée sur le support d'immobilisation représenté par la formule générale (3) est cyanuré à l'aide d'un réactif de cyanuration pour le convertir en un résidu cyanocystéine,
produisant ainsi une protéine immobilisée représentée par la formule générale (4) :
NH₂-R₁-CO-NH-R₂-CO-NH-Y (4)
dans laquelle R₁ et R₂ représentent des séquences d'acides aminés arbitraires, R₃ représente une séquence d'acides aminés qui a une charge négative forte autour de la région neutre et peut acidifier le point isoélectrique de NH₂-R₁-CONH-R₂-CO-NH-CH(CH₂-SH)-CO-NH-R₃-COOH et Y représente un support d'immobilisation arbitraire.

2. Procédé de production d'une protéine immobilisée selon la revendication 1, dans lequel la protéine représentée par la formule générale (2) comporte un groupe sulfhydryle représenté par NH₂-R₁-CONH-R₂-CO-NH-CH(CH₂-SH)-CO-NH-CH(CH₃)-CO-[NH-CH(CH₂-COOH)-CO]ₙ-OH, dans laquelle R₁ et R₂ représentent des séquences d'acides aminés arbitraires et n est un nombre naturel.

3. Procédé de production d'une protéine immobilisée selon la revendication 1 ou 2, dans lequel R₂ dans la protéine représentée par la formule générale (2) est la polyglycine.

4. Procédé de production d'une protéine immobilisée selon l'une quelconque des revendications 1 à 3, dans lequel R₁ dans la protéine représentée par la formule générale (2) est une enzyme.

5. Procédé de production d'un réseau de protéines par immobilisation sur un substrat d'immobilisation, d'une protéine représentée par la formule (1) :
NH₂-R₁-COOH (1)
dans laquelle une protéine comportant un groupe sulfhydryle représenté par la formule générale (2) est produite :
NH₂-R₁-CONH-R₂-CO-NH-CH(CH₂-SH)-CO-NH-R₃-COOH (2)
la protéine résultante est immobilisée par adsorption sur le substrat d'immobilisation représenté par la formule générale (3) tout en formant le réseau :
NH₂-Y (3),
et
un groupe sulfhydryle du résidu cystéine dans la protéine représentée par la formule générale (2) qui a été adsorbée sur le support d'immobilisation représenté par la formule générale (3) est cyanuré à l'aide d'un réactif de cyanuration pour le convertir en un résidu cyanocystéine.
produisant ainsi un réseau de protéines comportant une protéine immobilisée représentée par la formule générale (4) :
NH₂-R₁-CO-NH-R₂-CO-NH-Y (4)
dans laquelle R₁ et R₂ représentent des séquences d'acides aminés arbitraires, R₃ représente une séquence d'acides aminés qui a une charge négative forte autour de la région neutre et peut acidifier le point isoélectrique de NH₂-R₁-CONH-R₂-CO-NH-CH(CH₂-SH)-CO-NH-R₃-COOH et Y représente un support d'immobilisation arbitraire.

6. Procédé de production d'un réseau de protéines selon la revendication 5, dans lequel le procédé pour adsorber la protéine sur un substrat d'immobilisation tout en formant le réseau est réalisé par un système de jet d'encre.

7. Procédé de production d'un réseau de protéines selon la revendication 6, dans lequel un réactif de cyanuration, qui cyanure un groupe sulfhydryle du résidu cystéine, est laissé agir à l'aide d'une imprimante à jet d'encre.

8. Procédé de production d'un réseau de protéines selon l'une quelconque des revendications 5 à 7, dans lequel la protéine représentée par la formule générale (2) comporte un groupe sulfhydryle représenté par NH₂-R₁-CONH-R₂-CO-NH-CH(CH₂-SH)-CO-NH-CH(CH₃)-CO-[NH-CH(CH₂-COOH)-CO]ₙ-OH, dans laquelle R₁ et R₂ représentent des séquences d'acides aminés arbitraires et n est un nombre naturel.
